# EUROPEAN PATENT SPECIFICATION

(11) **EP 2 794 927 B1**
(45) Date of publication and mention of the grant of the patent: **12.04.2017**
(21) Application number: 12859854.7
(22) Date of filing: 21.12.2012
(51) Int. Cl.: C12Q 1/68, C12P 19/34

(54) **AMPLIFICATION PRIMERS AND METHODS**
AMPLIFIKATIONSPRIMER UND VERFAHREN
AMORCES D'AMPLIFICATION ET PROCÉDÉS ASSOCIÉS

(30) Priority: 22.12.2011 US 201161578976 P
(43) Date of publication of application: 29.10.2014
(73) Proprietor: Ibis Biosciences, Inc., Carlsbad, CA 92008 (US)
(72) Inventor: GRAY, Phillip N., Carlsbad California 92011 (US); ESHOO, Mark W., San Diego California 92130 (US)
(74) Representative: Chapman, Desmond Mark
(86) International application number: PCT/US2012/071308
(87) International publication number: WO 2013/096802

(56) References cited:
- EP-A1- 1 103 624
- WO-A1-2011/151777
- US-A1- 2002 172 965
- US-A1- 2003 143 536
- US-A1- 2004 180 372
- US-A1- 2006 292 611
- Qiagen: "QuantiTect® Whole Transcriptome Handbook", , July 2011 (2011-07), pages 1-28, XP002741376, Retrieved from the Internet: URL:https://www.qiagen.com/gb/resources/do wnload [retrieved on 2015-06-25]
- SHOAIB ET AL.: 'Multiple displacement amplification for complex mixtures of DNA fragments.' BMC GENOMICS vol. 9, no. 415, 15 September 2008, pages 1 - 14, XP021042155
- DONALDSON ET AL.: 'Metagenomic Analysis of the Viromes of Three North American Bat Species: Viral Diversity among Different Bat Species That Share a Common Habitat.' JOURNAL OF VIROLOGY. vol. 84, no. 24, December 2010, pages 13004 - 13018, XP055076468
- BROOKS ET AL.: 'Whole Genome Methylation Scanning Based on phi29 Polymerase Amplification.' BIOCHEMISTRY (MOSCOW). vol. 76, no. 9, 03 April 2011, pages 999 - 1002, XP055076469

## Description

### FIELD OF THE INVENTION

The present invention relates to methods for performing amplification (e.g., whole genome amplification) employing primers that have a 5' restriction site, a 3' random sequence (e.g., a random hexamer), and an identifiable barcode sequence.

### BACKGROUND

In many fields of research such as genetic diagnosis, cancer research or forensic medicine, the scarcity of genomic DNA can be a severely limiting factor on the type and quantity of genetic tests that can be performed on a sample. One approach designed to overcome this problem is whole genome amplification. The objective is to amplify a limited DNA sample in a non-specific manner in order to generate a new sample that is indistinguishable from the original but with a higher DNA concentration. The aim of a typical whole genome amplification technique is to amplify a sample up to a microgram level while respecting the original sequence representation.

The first whole genome amplification methods were described in 1992, and were based on the principles of the polymerase chain reaction. Zhang and coworkers (Zhang, L., et al. Proc. Natl. Acad. Sci. USA, 1992, 89: 5847-5851) developed the primer extension PCR technique (PEP) and Telenius and collaborators (Telenius et al., Genomics. 1992, 13(3):718-25) designed the degenerate oligonucleotide-primed PCR method (DOP-PCR). PEP involves a high number of PCR cycles, generally using Taq polymerase and 15 base random primers that anneal at a low stringency temperature. DOP-PCR is a method which generally uses Taq polymerase and semi-degenerate oligonucleotides that bind at a low annealing temperature at approximately one million sites within the human genome. The first cycles are followed by a large number of cycles with a higher annealing temperature, allowing only for the amplification of the fragments that were tagged in the first step.

Multiple displacement amplification (MDA, also known as strand displacement amplification; SDA) is a non-PCR-based isothermal method based on the annealing of random hexamers to denatured DNA, followed by strand-displacement synthesis at constant temperature (Blanco et al., 1989, J. Biol. Chem. 264:8935-40; Dean, F.B. et al. (2002) Comprehensive human genome amplification using multiple displacement amplification; Proc. Natl. Acad. Sci. USA 99,5261; and Van, J. et al. (2004) Assessment of multiple displacement amplification in molecular epidemiology. Biotechniques 37, 136). It has been applied to small genomic DNA samples, leading to the synthesis of high molecular weight DNA with limited sequence representation bias (Lizardi et al., Nature Genetics 1998, 19, 225-232; Dean et al., Proc. Natl. Acad. Sci. U.S.A. 2002, 99, 5261-5266). As DNA is synthesized by strand displacement, a gradually increasing number of priming events occur, forming a network of hyper-branched DNA structures. The reaction can be catalyzed by the Phi29 DNA polymerase or by the large fragment of the Bst DNA polymerase. The Phi29 DNA polymerase possesses a proofreading activity resulting in error rates 100 times lower than the Taq polymerase. MDA type methods, however, require many hours (e.g., 6 hours) to generate a sufficient fold amplification.

WO 2011/151777 (Tullo et al.) relates to a method for preparing and amplifying representative cDNA libraries for next-generation high throughput sequencing, and to kits and cartridges for automation kits which use the method.

### SUMMARY OF THE INVENTION

The invention provides a method of generating amplified nucleic acid from RNA comprising: (a) exposing an RNA template sequence to a set of primers under reverse transcription conditions such that a mixed population of cDNA first strands are generated, wherein said set of primers comprises individual primers each comprising: (i) a 5' restriction sequence site, (ii) a 3' random hexamer sequence, and (iii) a barcode sequence located between the 5' restriction sequence site and the 3' random hexamer sequence, wherein said 5' restriction sequence site in each of said individual primers is identical and wherein said set of primers comprises every or nearly every possible random hexamer sequence, and wherein each of said cDNA first strands have one of said individual primers at its 5' terminus; (b) exposing said mixed population of cDNA first strands to said set of primers under polymerization conditions such that a mixed population of double-stranded cDNA molecules is generated, (c) digesting said mixed population of double-stranded cDNA molecules with a restriction enzyme specific for said 5' restriction sequence site, (d) treating said mixed population of double-stranded cDNA molecules with a ligating agent such that individual double-stranded cDNA molecules are ligated to each other to form a mixed population of concatamers; (e) exposing said mixed population of concatamers to random primers under whole genome amplification conditions such that amplified nucleic acid is generated; (f) at least partially digesting said amplified nucleic acid with the same restriction enzyme specific for said 5' restriction sequence site thereby generating a plurality of digested sequences; and (g) ligating sequencing adapter sequences to the ends of said plurality of digested sequences to generate a mixed population of adapter-ligated sequencing templates, wherein said sequencing adapter sequences contain a restriction enzyme site that is identical to the 5' restriction sequence site in the set of primers.

Disclosed herein are methods, compositions, and kits for performing amplification (e.g., whole genome amplification) employing primers that have a 5' restriction site, a 3' random sequence (e.g., a random hexamer), and an identifiable barcode sequence. In certain embodiments, the amplification generates individual amplified sequenced that are ligated together to form concatamers containing at least two amplified sequences (e.g., not contiguous on the original target sequence) that are separated by the barcode sequences. In particular embodiments, a plurality of the concatamers are sequenced and aligned with an alignment algorithm that uses the barcode sequences to identify artificial junctions between amplified sequences.

In some embodiments, there are disclosed herein methods of generating amplified nucleic acid from RNA comprising: a) exposing an RNA template sequence to a set of primers under reverse transcription conditions such that a mixed population of cDNA first strands are generated, wherein the set of primers comprises individual primers each comprising: i) a 5' restriction sequence site, ii) a 3' random sequence (e.g., pentamer sequence, hexamer sequence, or longer random sequence) and iii) a barcode sequence (e.g., 2-15 identifiable base sequence, or 5-10 base identifiable sequence), wherein the set of primers comprises every or nearly every possible random pentamer, hexamer, or longer sequence, and wherein each of the cDNA first strands have one of the individual primers at its 5' terminus; b) exposing the mixed population of cDNA first strands to the set of primers under polymerization conditions such that a mixed population of double-stranded cDNA molecules is generated, c) digesting the mixed population of double-stranded cDNA molecules with a restriction enzyme specific for the 5' restriction sequence site, d) treating the mixed population of double-stranded cDNA molecules with a ligating agent such that individual double-stranded cDNA molecules are ligated to each other to form a mixed population of concatamers; and e) exposing the mixed population of concatamers to random primers under whole genome amplification conditions such that amplified nucleic acid is generated. In certain embodiments, the concatamers contain two, three, four, five, six, or more of the individual double-stranded cDNA molecules.

In certain embodiments, the RNA template is less than 2000 bases in length. In further embodiments, the mixed population of concatamers comprise individual concatamers that are about 2000 bases in length or longer. In additional embodiments, the sequencing adapter sequences contain a restriction enzyme site that is identical to the 5' restriction sequence site in the primers. In further embodiments, the methods further comprise at least partially digesting the amplified nucleic acid with a restriction enzyme specific for the 5' restriction sequence site thereby generating a plurality of digested sequences.

In other embodiments, the methods further comprise ligating sequencing adapter sequences to the ends of the plurality of digested sequences to generate a mixed population of adapter-ligated sequencing templates. In certain embodiments, the sequencing adapter sequences contain a restriction enzyme site. In particular embodiments, the restriction site in the sequencing adapters is the same as the restriction site present in the primers. In other embodiments, the sequencing adapter sequences are hairpin sequences.

In some embodiments, the plurality of digested sequences comprise individual digested sequences that each contain the base sequences of only one of the double-stranded cDNA molecules from the mixed population of double-stranded cDNA molecules. In other embodiments, the plurality of digested sequences comprise individual digested sequences that each contain the base sequences of two or more of the double-stranded cDNA molecules from the mixed population of double-stranded cDNA molecules, wherein the base sequences are separated from each other by the bar code sequences (e.g., if there are two sequences, there is one bar code separating them; if there are three sequences (or more) there is a bar code sequence between each sequence). In particular embodiments, the methods further comprise sequencing at least one of the individual digested sequences to generate electronic sequence information, and processing the electronic sequence information with an alignment algorithm wherein the bar code sequences are used to identify artificial junctions between the base sequences of two or more of the double-stranded cDNA molecules.

In certain embodiments, the sequencing is accomplished by a method selected from the group consisting of: Sanger dideoxy sequencing, 454-pyrosequencing, Solexa/Illumina sequencing, Helicos true molecule sequencing, Pacific Biosciences SMRT sequencing, or Ion Torrent sequencing. In particular embodiments, the 5' restriction sequence site in each of the individual primers is identical. In further embodiments, the barcode sequence in each of the individual primers is identical.

In some embodiments, there are disclosed herein methods of generating amplified nucleic acid from DNA comprising: a) treating a mixed population of DNA template sequences with a ligating agent such that individual DNA template sequences (e.g., two, or three, or four, or more) are ligated to each other to form a mixed population of concatamers, wherein the mixed population of DNA template sequences comprises different individual DNA template sequences; and b) exposing the concatamers to a set of primers under whole genome amplification conditions such that a mixed population of amplified double-stranded DNA molecules is generated, wherein the set of primers comprises individual primers each comprising: i) a 5' restriction sequence site, ii) a 3' random sequence (e.g., pentamer or hexamer or longer sequence), and iii) a barcode sequence, wherein the set of primers comprises every or nearly every possible random pentamer or hexamer sequence (e.g., every or 90% ... 95% ... 99% ... of every possible random hexamer).

In certain embodiments, the methods further comprise: c) at least partially digesting the mixed population of amplified double-stranded DNA molecules with a restriction enzyme specific for the 5' restriction sequence site thereby generating a plurality of digested sequences. In further embodiments, the methods further comprise: d) ligating sequencing adapter sequences to the ends of the plurality of digested sequences to generate a mixed population of adapter-ligated sequencing templates. In other embodiments, the different individual DNA template sequences are less than 2000 bases in length (e.g., less than 2000 bases, less than 1500 bases, less than 1000 bases, less than 500 bases, less than 250 bases, or less than 150 bases; or between 100-1000 bases or between 250-1500 bases). In further embodiments, the mixed population of concatamers comprise individual concatamers that are about 2000 bases in length or longer (e.g., 2000 bases ... 2500 bases ... 3000 bases ... 4000 bases or longer). In other embodiments, the sequencing adapter sequences contain a restriction enzyme site (e.g., the same site as present in the primers). In certain embodiments, the sequencing adapter sequences are hairpin sequences.

In particular embodiments, the plurality of digested sequences comprise individual digested sequences that each contain the base sequences of only one of the different individual DNA template sequences. In other embodiments, the plurality of digested sequences comprise individual digested sequences that each contain the base sequences of two or more (e.g., 2, 3, 4, 5, 6, or more) of the different individual DNA template sequences, wherein the base sequences are separated from each other by the bar code sequences. In further embodiments, the mixed population of adapter-ligated sequencing templates comprises individual adapter-ligated sequencing templates, where the method further comprises sequencing at least one (or most or all) of the individual adapter-ligated sequencing templates to generate electronic sequence information, and processing the electronic sequence information with an alignment algorithm wherein the bar code sequences are used to identify artificial junctions between the bases sequences of two or more of the individual DNA template sequences. In certain embodiments, the 5' restriction sequence site in each of the individual primers is identical. In additional embodiments, the barcode sequence in each of the individual primers is identical.

In some embodiments, there are disclosed herein compositions comprising a set of primers, wherein the set of primers comprises individual primers each comprising: i) a 5' restriction sequence site, ii) a 3' random pentamer, hexamer, or longer sequence, and iii) a barcode sequence, wherein the set of primers comprises every or nearly every possible random pentamer, hexamer, or longer sequence. In certain embodiments, the 5' restriction sequence site in each of the individual primers is identical. In other embodiments, the barcode sequence in each of the individual primers is identical.

In some embodiments, there are disclosed herein kits and systems comprising: a) a composition comprising a set of primers, wherein the set of primers comprises individual primers each comprising: i) a 5' restriction sequence site, ii) a 3' random pentamer, hexamer, or longer sequence, and iii) an bar code sequence, wherein the set of primers comprises every or nearly every possible random pentamer, hexamer, or longer sequence; and b) a polymerase suitable for performing whole genome amplification. In certain embodiments, the polymerase comprises Phi29 or the large fragment of the Bst DNA polymerase, or similarly functioning enzyme.

In certain embodiments, there are disclosed herein methods of generating sequence alignments comprising: a) sequencing a mixed population of concatamers to generate sequence information, wherein the mixed population of concatamers comprises a plurality of individual concatamers each comprising: i) at least two different library sequences from the genome of an organism, wherein the at least two different library sequences are not contiguous in the genome; and ii) at least one bar code sequence located between the at least two different library sequences; and b) inputting the sequence information into a system, wherein the system comprises: i) a computer processor for receiving, processing, and communicating data, ii) a computer program, embedded within the computer processor, which is configured to process the sequence information to form sequence alignments; c) processing the sequence information with the computer program such that the bar code sequences are used to form sequence alignments by identifying artificial junctions between the at least two different library sequences; and d) communicating the outcome from the computer program to a user. In particular embodiments, the methods further comprise a step before step a) of generating the mixed population of concatamers by whole genome amplification and ligation of whole genome amplification products.

In some embodiments, there are disclosed herein compositions comprising: a set of library sequences, wherein said set of library sequences comprises individual library sequences that comprise: i) at least one bar code sequence; ii) at least two DNA inserts, wherein each of said DNA inserts is an amplified portion of a target sequence, and wherein said at least two DNA inserts are separated by one of said bar codes sequences; iii) a restriction sequence site, iv) a random pentamer, hexamer sequence or longer sequence, and v) adapter sequences (e.g., one at each end), wherein said set of library sequences comprises every or nearly every sequence from a target sequence. In certain embodiments, the restriction sequence site is adjacent to said at least one barcode sequence. In further embodiments, the random pentamer or hexamer, or longer random sequence is adjacent to the at least one barcode sequence. In further embodiments, the target sequence is over 2000 bases in length. In additional embodiments, the target sequence is over 20,000 bases in length.

The present methods are not limited by the endonuclease restriction enzyme site or enzyme that is employed. In certain embodiments, the restriction site is recognized by, or the enzyme employed, is: BamH1, EcoR1, EcoRII, HindII, HindIII, HinfI, HpaI, MspI, and SmaI. Many other restriction sites and enzymes are well known in the art.

### DESCRIPTION OF THE FIGURES

Figures 1A-C show an exemplary flow diagram of employing the primers described herein in to amplify RNA, and, in combination with WGA amplification and product ligation, to generate a primer adapted sequencing library.

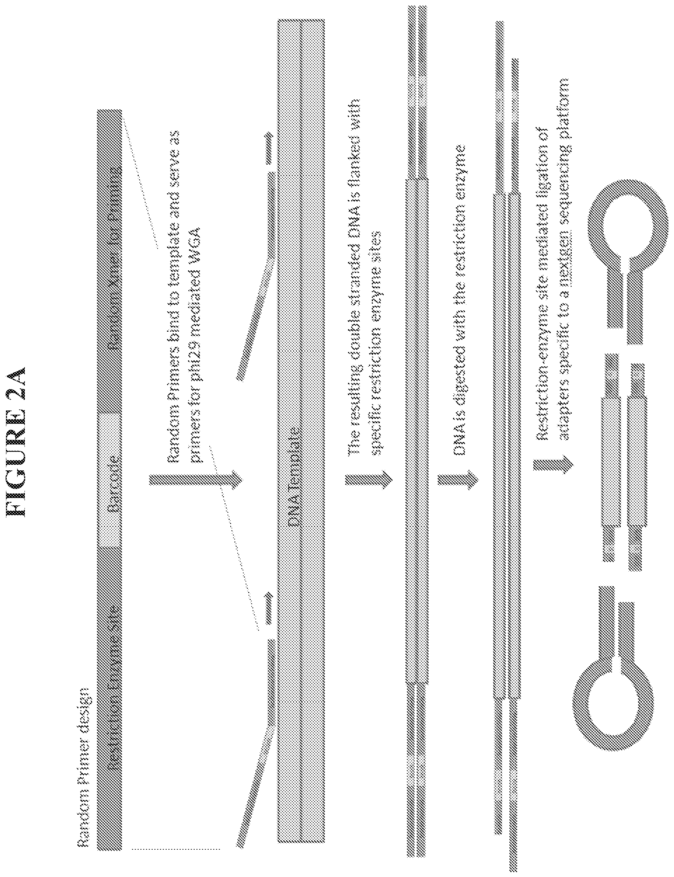

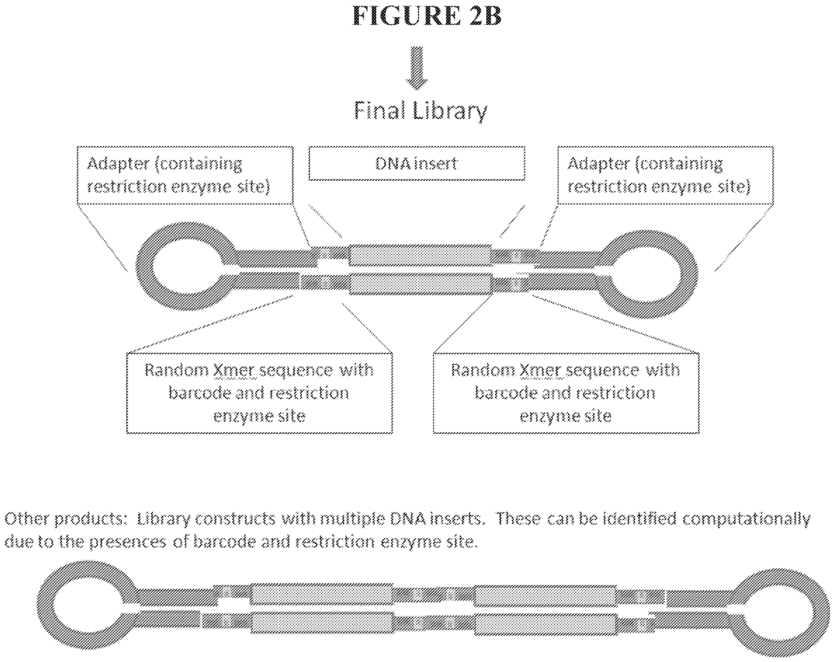

Figures 2A-B show an exemplary flow diagram of employing the primers described herein to amplify DNA and generate a primer adapted sequencing library.

### DETAILED DESCRIPTION

There are disclosed herein methods, compositions, and kits for performing amplification (e.g., whole genome amplification) employing primers that have a 5' restriction site, a 3' random sequence (e.g., a random hexamer), and an identifiable barcode sequence. In certain embodiments, the amplification generates individual amplified sequenced that are ligated together to form concatamers containing at least two amplified sequences (e.g., not originally contiguous on the original target sequence) that are separated by the barcode sequences. In particular embodiments, a plurality of the concatamers are sequenced and aligned with an alignment algorithm that uses the barcode sequences to identify artificial junctions between amplified sequences. In particular embodiments, there are disclosed herein methods and compositions for library preparation of short RNA and DNA templates for next generation sequencing using whole genome amplification and restriction enzymes.

Whole genome amplification (WGA) of RNA viruses (or other small ssRNA molecules) using Phi29 has been described in the literature as a very inefficient process. It has been reported that Phi29 DNA polymerase cannot amplify RNA or small cDNA fragments less than 2000 bases generated from reverse transcriptase. One approach to this problem is described by Berthet et al., BMC Molecular Biology, 2008, 9:77, which generated cDNA fragments of RNA viruses using random hexamers and ligated the double stranded cDNA fragments in a random manner via blunt end ligation. The concatenated cDNA molecules were used as a substrate for Phi29. The main disadvantage of this approach is that it creates chimeric DNA fragments with artificial junctions in the WGA library. These chimeric DNA fragments pose a challenge for alignment algorithms created for next generation sequencing data and typically are discarded during the alignment process. The present method incorporates specific DNA sequences in the concatenated DNA, allowing easy identification of the artificial junctions. As a result, individual sequence reads from the concatenated DNA molecule may be recovered.

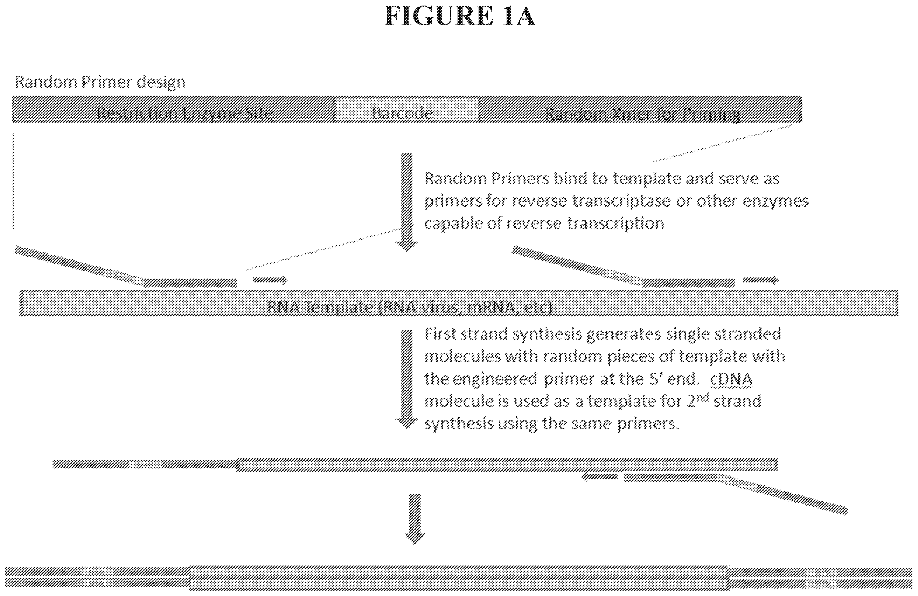

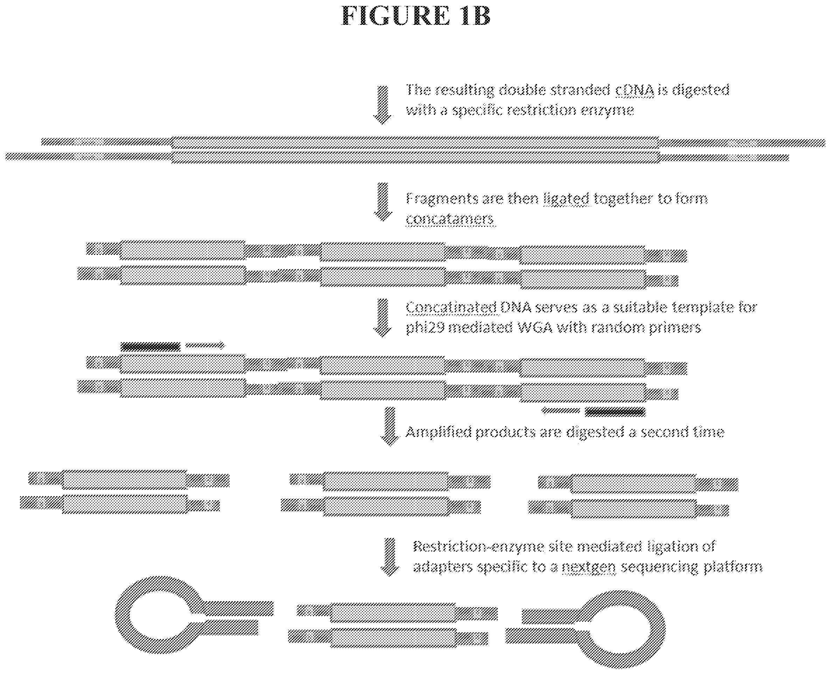

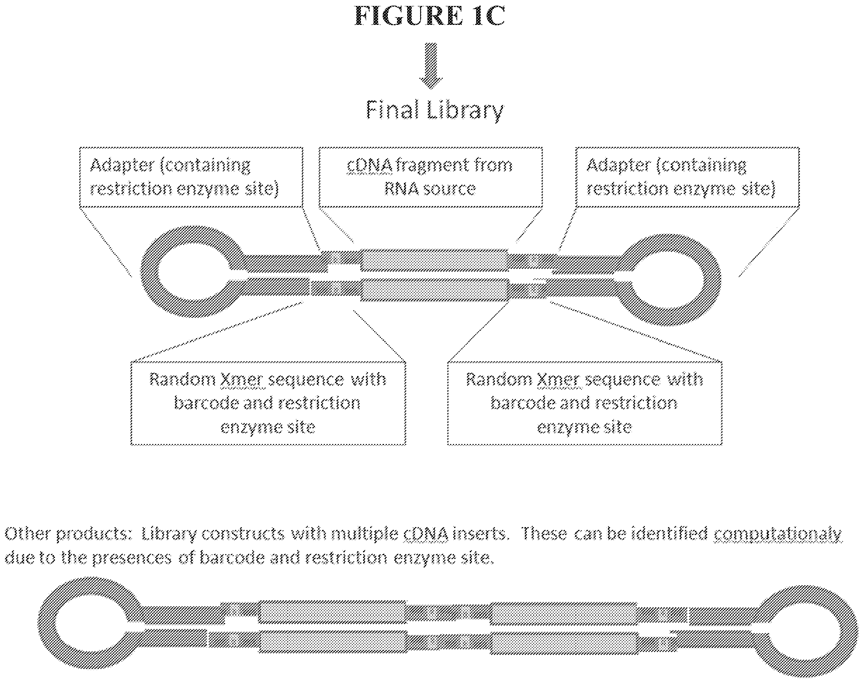

In certain embodiments, there are disclosed herein methods that use random priming of target nucleic acid using primers containing a restriction enzyme site at the 5' end. For RNA, double stranded cDNA molecules that result from the random primers and reverse transcriptase (or other polymerase capable of reverse transcription), followed by second strand synthesis, are digested with a restriction enzyme that will cut the ends of the random hexamers and create cDNA molecules with a specific restriction enzyme site at the 5' and 3' end of each molecule (see, e.g., Figure 1). These cDNA molecules are then ligated together to form large templates of concatenated cDNA for whole genome amplification. Following whole genome amplification, the amplified products are digested with the same restriction enzyme, producing cDNA fragments flanked with the restriction enzyme site. The random primers with the restriction site have a barcode sequence to distinguish them from a naturally occurring restriction site in the genome of interest. Next, adapters specific to a particular sequencing platform (e.g., Pacific Biosciences, Illumina, Ion Torrent, 454, SOLiD, etc.) are engineered to contain the restriction enzyme site that matches the site contained in the whole genome amplified cDNA library. The adapters that contain the restriction sites ("RE-adapters") are then mixed with the whole genome amplified cDNA fragments that contain the restriction sites ("RE-cDNA") and are ligated to yield a cDNA library flanked with the adapters and ready for sequencing (Adapter-RE-cDNA-RE-Adapter). This process can be utilized, for example, with DNA or RNA, or degraded nucleic acids from formalin-fixed, paraffin-embedded (FFPE) samples/tissues.

As indicated above, one of the main disadvantage of the prior art approach is that it creates artificial junctions in the WGA library that cannot be easily identified computationally. Next generation sequencing algorithms that align short reads are limited in their ability to process data with artificial junctions. In certain embodiments, the present method generates two types of products in the library. The first type contains random DNA fragments flanked by the RE-adapters, which do not contain artificial junctions. The second type contains multiple random DNA fragments flanked by the RE-adapters. These concatenated DNA fragments are separated by a known sequence (RE site with bar code followed by a random Xmer sequence) that can be easily identified computationally. As a result, the sequence read can be divided into its individual, non-concatinated reads.

In certain embodiments, the present method can be used to take unknown samples containing nucleic acid (e.g., either RNA or DNA) and produce templates for WGA using phi29 (or other WGA polymerases). Adapters specific for next generation sequencing (or sequencing by synthesis) platforms (Pacific Biosciences, Illumina, Ion Torrent, etc) may be ligated to the library and sequence data obtained from trace amounts of input.

Barcode sequences are used in certain primers of the present method. The barcode sequence can be any identifiable sequence located between the restriction enzyme site and random hexamer sequence. Generally, these sequences are about 5-10 nucleotides in length. Their purpose is to distinguish the restriction enzyme sites introduced from priming with the random Xmer/barcode/restriction enzyme oligos from restriction enzyme sites that occur naturally in the target genome (which would produce an artificial junction). DNA barcodes may vary widely in size and compositions. The following references provide guidance for selecting sets of oligonucleotide barcodes appropriate for particular embodiments: Brenner, U.S. Pat. No. 5,635,400; Brenner et al, Proc. Natl. Acad. Sci. 97:1665-1670 (2000), Shoemaker et al, Nature Genetics, 14:450-456 (1996); Morris et al, European patent publication 0799897A1; Wallace, U.S. Pat. No. 5,981,179; and the like. In different applications of the method, oligonucleotide barcodes can each have a length within a range of from 4 to 36 nucleotides, or from 6 to 30 nucleotides, or from 8 to 20 or 5 to 10 nucleotides, respectively.

In certain embodiments, the amplified sequences that are generated are sequenced. The present method is not limited by the sequencing technique employed. Exemplary sequencing methods are described below. Illustrative non-limiting examples of nucleic acid sequencing techniques include, but are not limited to, chain terminator (Sanger) sequencing, dye terminator sequencing, and next generation sequencing methods.

Chain terminator sequencing uses sequence-specific termination of a DNA synthesis reaction using modified nucleotide substrates. Extension is initiated at a specific site on the template DNA by using a short radioactive, or other labeled, oligonucleotide primer complementary to the template at that region. The oligonucleotide primer is extended using a DNA polymerase, standard four deoxynucleotide bases, and a low concentration of one chain terminating nucleotide, most commonly a di-deoxynucleotide. This reaction is repeated in four separate tubes with each of the bases taking turns as the di-deoxynucleotide. Limited incorporation of the chain terminating nucleotide by the DNA polymerase results in a series of related DNA fragments that are terminated only at positions where that particular di-deoxynucleotide is used. For each reaction tube, the fragments are size-separated by electrophoresis in a slab polyacrylamide gel or a capillary tube filled with a viscous polymer. The sequence is determined by reading which lane produces a visualized mark from the labeled primer as you scan from the top of the gel to the bottom.

Dye terminator sequencing alternatively labels the terminators. Complete sequencing can be performed in a single reaction by labeling each of the di-deoxynucleotide chain-terminators with a separate fluorescent dye, which fluoresces at a different wavelength.

A set of methods referred to as "next-generation sequencing" techniques have emerged as alternatives to Sanger and dye-terminator sequencing methods (Voelkerding et al., Clinical Chem., 55: 641-658, 2009; MacLean et al., Nature Rev. Microbiol., 7: 287-296). Next-generation sequencing technology allows for de novo sequencing of whole genomes to determine the primary nucleic acid sequence of an organism. Next-generation sequencing technology also provide targeted re-sequencing (deep sequencing) which allows for sensitive mutation detection within a population of wild-type sequence. Some examples include recent work describing the identification of HIV drug-resistant variants as well as EGFR mutations for determining response to anti-TK therapeutic drugs. Publications describing the next-generation sequencing permit the simultaneous sequencing of multiple samples during a typical sequencing run including, for example: Margulies, M. et al. "Genome Sequencing in Microfabricated High-Density Picolitre Reactors", Nature, 437, 376-80 (2005); Mikkelsen, T. et al. "Genome-Wide Maps of Chromatin State in Pluripotent and Lineage-Committed Cells", Nature, 448, 553-60 (2007); McLaughlin, S. et al. "Whole-Genome Resequencing with Short Reads: Accurate Mutation Discovery with Mate Pairs and Quality Values", ASHG Annual Meeting (2007); Shendure J. et al. "Accurate Multiplex Polony Sequencing of an Evolved Bacterial Genome", Science, 309, 1728-32 (2005); Harris, T. et al. "Single-Molecule DNA Sequencing of a Viral Genome", Science, 320, 106-9 (2008); Simen, B. et al. "Prevalence of Low Abundance Drug Resistant Variants by Ultra Deep Sequencing in Chronically HIV-infected Antiretroviral (ARV) Naive Patients and the Impact on Virologic Outcomes", 16th International HIV Drug Resistance Workshop, Barbados (2007); Thomas, R. et al. "Sensitive Mutation Detection in Heterogeneous Cancer Specimens by Massively Parallel Picoliter Reactor Sequencing", Nature Med., 12, 852-855 (2006); Mitsuya, Y. et al. "Minority Human Immunodeficiency Virus Type 1 Variants in Antiretroviral-Naive Persons with Reverse Transcriptase Codon 215 Revertant Mutations", J. Vir., 82, 10747-10755 (2008); Binladen, J. et al. "The Use of Coded PCR Primers Enables High-Throughput Sequencing of Multiple Homolog Amplification Products by 454 Parallel Sequencing", PLoS ONE, 2, e197 (2007); and Hoffmann, C. et al. "DNA Bar Coding and Pyrosequencing to Identify Rare HIV Drug Resistance Mutations", Nuc. Acids Res., 35, e91 (2007).

Compared to traditional Sanger sequencing, next-gen sequencing technology produces large amounts of sequencing data points. A typical run can easily generate tens to hundreds of megabases per run, with a potential daily output reaching into the gigabase range. This translates to several orders of magnitude greater than a standard 96-well plate, which can generate several hundred data points in a typical multiplex run. Target amplicons that differ by as little as one nucleotide can easily be distinguished, even when multiple targets from related species or organisms are present. This greatly enhances the ability to do accurate genotyping. Next-gen sequence alignment software programs used to produce consensus sequences can easily identify novel point mutations, which could result in new strains with associated drug resistance. The use of primer bar coding also allows multiplexing of different patient samples within a single sequencing run.

Next-generation sequencing (NGS) methods share the common feature of massively parallel, high-throughput strategies, with the goal of lower costs in comparison to older sequencing methods. NGS methods can be broadly divided into those that require template amplification and those that do not. Amplification-requiring methods include pyrosequencing commercialized by Roche as the 454 technology platforms (e.g., GS 20 and GS FLX), the Solexa platform commercialized by Illumina, and the Supported Oligonucleotide Ligation and Detection (SOLiD) platform commercialized by Applied Biosystems. Non-amplification approaches, also known as single-molecule sequencing, are exemplified by the HeliScope platform commercialized by Helicos BioSciences, Ion Torrent, and emerging platforms commercialized by VisiGen and Pacific Biosciences, respectively.

In pyrosequencing (Voelkerding et al., Clinical Chem., 55: 641-658, 2009; MacLean et al., Nature Rev. Microbiol., 7: 287-296; U.S. Pat. No. 6,210,891; U.S. Pat. No. 6,258,568), template DNA is fragmented, end-repaired, ligated to adaptors, and clonally amplified in-situ by capturing single template molecules with beads bearing oligonucleotides complementary to the adaptors. Each bead bearing a single template type is compartmentalized into a water-in-oil microvesicle, and the template is clonally amplified using a technique referred to as emulsion PCR. The emulsion is disrupted after amplification and beads are deposited into individual wells of a picotitre plate functioning as a flow cell during the sequencing reactions. Ordered, iterative introduction of each of the four dNTP reagents occurs in the flow cell in the presence of sequencing enzymes and luminescent reporter such as luciferase. In the event that an appropriate dNTP is added to the 3' end of the sequencing primer, the resulting production of ATP causes a burst of luminescence within the well, which is recorded using a CCD camera. It is possible to achieve read lengths greater than or equal to 400 bases, and 1x10⁶ sequence reads can be achieved, resulting in up to 500 million base pairs (Mb) of sequence.

In the Solexa/Illumina platform (Voelkerding et al., Clinical Chem., 55: 641-658, 2009; MacLean et al., Nature Rev. Microbiol., 7: 287-296; U.S. Pat. No. 6,833,246; U.S. Pat. No. 7,115,400; U.S. Pat. No. 6,969,488), sequencing data are produced in the form of shorter-length reads. In this method, single-stranded fragmented DNA is end-repaired to generate 5'-phosphorylated blunt ends, followed by Klenow-mediated addition of a single A base to the 3' end of the fragments. A--addition facilitates addition of T--overhang adaptor oligonucleotides, which are subsequently used to capture the template-adaptor molecules on the surface of a flow cell that is studded with oligonucleotide anchors. The anchor is used as a PCR primer, but because of the length of the template and its proximity to other nearby anchor oligonucleotides, extension by PCR results in the "arching over" of the molecule to hybridize with an adjacent anchor oligonucleotide to form a bridge structure on the surface of the flow cell. These loops of DNA are denatured and cleaved. Forward strands are then sequenced with reversible dye terminators. The sequence of incorporated nucleotides is determined by detection of post-incorporation fluorescence, with each fluor and block removed prior to the next cycle of dNTP addition. Sequence read length ranges from 36 nucleotides to over 50 nucleotides, with overall output exceeding 1 billion nucleotide pairs per analytical run.

Sequencing nucleic acid molecules using SOLiD technology (Voelkerding et al., Clinical Chem., 55: 641-658, 2009; MacLean et al., Nature Rev. Microbiol., 7: 287-296; U.S. Pat. No. 5,912,148; U.S. Pat. No. 6,130,073) also involves fragmentation of the template, ligation to oligonucleotide adaptors, attachment to beads, and clonal amplification by emulsion PCR. Following this, beads bearing template are immobilized on a derivatized surface of a glass flow-cell, and a primer complementary to the adaptor oligonucleotide is annealed. However, rather than utilizing this primer for 3' extension, it is instead used to provide a 5' phosphate group for ligation to interrogation probes containing two probe-specific bases followed by 6 degenerate bases and one of four fluorescent labels. In the SOLiD system, interrogation probes have 16 possible combinations of the two bases at the 3' end of each probe, and one of four fluors at the 5' end. Fluor color and thus identity of each probe corresponds to specified color-space coding schemes. Multiple rounds (usually 7) of probe annealing, ligation, and fluor detection are followed by denaturation, and then a second round of sequencing using a primer that is offset by one base relative to the initial primer. In this manner, the template sequence can be computationally re-constructed, and template bases are interrogated twice, resulting in increased accuracy. Sequence read length averages 35 nucleotides, and overall output exceeds 4 billion bases per sequencing run.

In certain embodiments, nanopore sequencing in employed (see, e.g., Astier et al., J Am Chem Soc. 2006 Feb. 8; 128(5):1705-10). The theory behind nanopore sequencing has to do with what occurs when the nanopore is immersed in a conducting fluid and a potential (voltage) is applied across it: under these conditions a slight electric current due to conduction of ions through the nanopore can be observed, and the amount of current is exceedingly sensitive to the size of the nanopore. If DNA molecules pass (or part of the DNA molecule passes) through the nanopore, this can create a change in the magnitude of the current through the nanopore, thereby allowing the sequences of the DNA molecule to be determined.

HeliScope by Helicos BioSciences (Voelkerding et al., Clinical Chem., 55: 641-658, 2009; MacLean et al., Nature Rev. Microbiol., 7: 287-296; U.S. Pat. No. 7,169,560; U.S. Pat. No. 7,282,337; U.S. Pat. No. 7,482,120; U.S. Pat. No. 7,501,245; U.S. Pat. No. 6,818,395; U.S. Pat. No. 6,911,345; U.S. Pat. No. 7,501,245) is the first commercialized single-molecule sequencing platform. This method does not require clonal amplification. Template DNA is fragmented and polyadenylated at the 3' end, with the final adenosine bearing a fluorescent label. Denatured polyadenylated template fragments are ligated to poly(dT) oligonucleotides on the surface of a flow cell. Initial physical locations of captured template molecules are recorded by a CCD camera, and then label is cleaved and washed away. Sequencing is achieved by addition of polymerase and serial addition of fluorescently-labeled dNTP reagents. Incorporation events result in fluor signal corresponding to the dNTP, and signal is captured by a CCD camera before each round of dNTP addition. Sequence read length ranges from 25-50 nucleotides, with overall output exceeding 1 billion nucleotide pairs per analytical run.

Other emerging single molecule sequencing methods include real-time sequencing by synthesis using a VisiGen platform (Voelkerding et al., Clinical Chem., 55: 641-658, 2009; U.S. Pat. No. 7,329,492; U.S. patent application Ser. No. 11/671,956; U.S. patent application Ser. No. 11/781,166) in which immobilized, primed DNA template is subjected to strand extension using a fluorescently-modified polymerase and florescent acceptor molecules, resulting in detectible fluorescence resonance energy transfer (FRET) upon nucleotide addition.

Another real-time single molecule sequencing system developed by Pacific Biosciences (Voelkerding et al., Clinical Chem., 55: 641-658, 2009; MacLean et al., Nature Rev. Microbiol., 7: 287-296; U.S. Pat. No. 7,170,050; U.S. Pat. No. 7,302,146; U.S. Pat. No. 7,313,308; U.S. Pat. No. 7,476,503) utilizes reaction wells 50-100 nm in diameter and encompassing a reaction volume of approximately 20 zeptoliters (10x10⁻²¹ L). Sequencing reactions are performed using immobilized template, modified phi29 DNA polymerase, and high local concentrations of fluorescently labeled dNTPs. High local concentrations and continuous reaction conditions allow incorporation events to be captured in real time by fluor signal detection using laser excitation, an optical waveguide, and a CCD camera.

Ion torrent sequencing is a method of DNA sequencing based on the detection of hydrogen ions that are released during the polymerization of DNA. This is a method of "sequencing by synthesis," during which a complementary strand is built based on the sequence of a template stand. A microwell containing a template DNA strand to be sequenced is flooded with a single species of deoxyribonucleotide (dNTP). If the introduced dNTP is complementary to the leading template nucleotide, it is incorporated into the growing complementary strand. This causes the release of a hydrogen ion that triggers a hypersensitive ion sensor, which indicates that a reaction has occurred. If homopolymer repeats are present in the template sequence, multiple dNTP molecules will be incorporated in a single cycle. This leads to a corresponding number of released hydrogens and a proportionally higher electronic signal.

Various modification and variation of the described methods and compositions of the invention will be apparent to those skilled in the art. Although the invention has been described in connection with specific preferred embodiments, it should be understood that the invention as claimed should not be unduly limited to such specific embodiments.

## Claims

1. A method of generating amplified nucleic acid from RNA comprising:
a) exposing an RNA template sequence to a set of primers under reverse transcription conditions such that a mixed population of cDNA first strands are generated,
wherein said set of primers comprises individual primers each comprising: i) a 5' restriction sequence site, ii) a 3' random hexamer sequence, and iii) a barcode sequence located between the 5' restriction sequence site and the 3' random hexamer sequence, wherein said 5' restriction sequence site in each of said individual primers is identical and wherein said set of primers comprises every or nearly every possible random hexamer sequence, and
wherein each of said cDNA first strands have one of said individual primers at its 5' terminus;
b) exposing said mixed population of cDNA first strands to said set of primers under polymerization conditions such that a mixed population of double-stranded cDNA molecules is generated,
c) digesting said mixed population of double-stranded cDNA molecules with a restriction enzyme specific for said 5' restriction sequence site,
d) treating said mixed population of double-stranded cDNA molecules with a ligating agent such that individual double-stranded cDNA molecules are ligated to each other to form a mixed population of concatamers;
e) exposing said mixed population of concatamers to random primers under whole genome amplification conditions such that amplified nucleic acid is generated;
f) at least partially digesting said amplified nucleic acid with the same restriction enzyme specific for said 5' restriction sequence site thereby generating a plurality of digested sequences; and
g) ligating sequencing adapter sequences to the ends of said plurality of digested sequences to generate a mixed population of adapter-ligated sequencing templates, wherein said sequencing adapter sequences contain a restriction enzyme site that is identical to the 5' restriction sequence site in the set of primers.

2. The method of Claim 1, wherein said sequencing adapter sequences are hairpin sequences.

3. The method of Claim 1, wherein said plurality of digested sequences comprise individual digested sequences that each contain the base sequences of only one of said double-stranded cDNA molecules from said mixed population of double-stranded cDNA molecules.

4. The method of Claim 1, wherein said plurality of digested sequences comprise individual digested sequences that each contain the base sequences of two or more of said double-stranded cDNA molecules from said mixed population of double-stranded cDNA molecules, wherein said base sequences are separated from each other by said bar code sequences.

5. The method of Claim 4, further comprising sequencing at least one of said individual digested sequences to generate electronic sequence information, and processing said electronic sequence information with an alignment algorithm wherein said bar code sequences are used to identify artificial junctions between said base sequences of two or more of said double-stranded cDNA molecules.

6. The method of Claim 1, wherein said barcode sequence in each of said individual primers is identical.

7. The method of Claim 1, wherein said RNA template is less than 2000 bases in length.

8. The method of Claim 1, wherein said mixed population of concatamers comprise individual concatamers that are about 2000 bases in length or longer.

## Patentansprüche

1. Verfahren zum Erzeugen amplifizierter Nukleinsäure aus RNA, umfassend:
a) Aussetzen einer RNA-Matrizensequenz gegenüber einem Satz Primer unter Bedingungen einer reversen Transkription, so dass eine gemischte Population von Erststrang-cDNAs erzeugt wird,
wobei der Satz Primer einzelne Primer umfasst, die jeweils umfassen: i) eine 5'-Restriktionssequenzstelle, ii) eine statistische 3'-Hexamersequenz, und iii) eine Barcodesequenz, die sich zwischen der 5'-Restriktionssequenzstelle und der statistischen 3'-Hexamersequenz befindet, wobei die 5'-Restriktionssequenzstelle in jedem der einzelnen Primer identisch ist, und wobei der Satz Primer jede oder fast jede mögliche statistische Hexamersequenz umfasst, und
worin jede der Erststrang-cDNAs einen der einzelnen Primer an ihrem 5'-Terminus aufweist;
b) Aussetzen der gemischten Population von Erststrang-cDNAs gegenüber dem Satz Primer unter Polymerisationsbedingungen, so dass eine gemischte Population von doppelsträngigen cDNA-Molekülen erzeugt wird,
c) Spalten der gemischten Population von doppelsträngigen cDNA-Molekülen mit einem Restriktionsenzym, das für die 5'-Restriktionssequenzstelle spezifisch ist,
d) Behandeln der gemischten Population von doppelsträngigen cDNA-Molekülen mit einem Ligationsmittel, so dass die einzelnen doppelsträngigen cDNA-Moleküle miteinander ligiert werden, so dass eine gemischte Population von Concatemeren erzeugt wird;
e) Aussetzen der gemischten Population von Concatemeren gegenüber statistischen Primern unter Vollgenom-Amplifikationsbedingungen, so dass amplifizierte Nukleinsäure erzeugt wird;
f) zumindest partielles Spalten der amplifizierten Nukleinsäure mit dem gleichen Restriktionsenzym, das für die 5'-Restriktionssequenzstelle spezifisch ist, wodurch eine Anzahl von gespaltenen Sequenzen erzeugt wird; und
g) Ligieren von Sequenzierungsadaptersequenzen an die Enden der Anzahl von gespaltenen Sequenzen, so dass eine gemischte Population von Adapter-ligierten Sequenzierungsmatrizen erzeugt wird, wobei die Sequenzierungsadaptersequenzen eine Restriktionsenzymstelle enthalten, die zu der Restriktionssequenzstelle in dem Satz Primer identisch ist.

2. Verfahren nach Anspruch 1, wobei die Sequenzierungsadaptersequenzen Haarnadel-Sequenzen sind.

3. Verfahren nach Anspruch 1, wobei die Anzahl von gespaltenen Sequenzen einzelne gespaltene Sequenzen umfasst, die jeweils die Basensequenzen von nur einem der doppelsträngigen cDNA-Moleküle aus der gemischten Population von doppelsträngigen cDNA-Molekülen enthalten.

4. Verfahren nach Anspruch 1, wobei die Anzahl von gespaltenen Sequenzen einzelne gespaltene Sequenzen umfasst, die jeweils die Basensequenzen von zwei oder mehr der doppelsträngigen cDNA-Moleküle aus der gemischten Population von doppelsträngigen cDNA-Molekülen enthalten, wobei die Basensequenzen durch die Barcodesequenzen voneinander getrennt sind.

5. Verfahren nach Anspruch 4, zudem umfassend das Sequenzieren von zumindest einer der einzelnen gespaltenen Sequenzen, so dass eine elektronische Sequenzinformation erzeugt wird, und Verarbeiten der elektronischen Sequenzinformation mit einem Alignment-Algorithmus, bei dem die Barcodesequenzen verwendet werden, um künstliche Verbindungen zwischen den Basensequenzen von zwei oder mehr der doppelsträngigen cDNA-Moleküle zu identifizieren.

6. Verfahren nach Anspruch 1, wobei die Barcodesequenz in jedem der einzelnen Primer identisch ist.

7. Verfahren nach Anspruch 1, wobei die RNA-Matrize weniger als 2000 Basen lang ist.

8. Verfahren nach Anspruch 1, wobei die gemischte Population von Concatemeren einzelne Concatemere umfasst, die etwa 2000 Basen lang oder länger sind.

## Revendications

1. Procédé de génération d'acide nucléique amplifié à partir d'ARN, comprenant :
a) l'exposition d'une séquence gabarit d'ARN à un jeu d'amorces en conditions de transcription inverse, de manière à générer une population mixte de premiers brins de cADN,
ledit jeu d'amorces comprenant des amorces individuelles, chacune comprenant :
i) un site de séquence de restriction 5',
ii) une séquence hexamère aléatoire 3', et
iii) une séquence code-barres située entre le site de séquence de restriction 5' et la séquence hexamère aléatoire 3',
ledit site de séquence de restriction 5' étant identique dans chacune desdites amorces individuelles, et ledit jeu d'amorces comprenant toutes ou presque toutes les séquences hexamères aléatoires possibles, et
chacun desdits premiers brins de cADN comportant une desdites amorces individuelles à son extrémité 5' ;
b) l'exposition de ladite population mixte de premiers brins de cADN audit jeu d'amorces en conditions de polymérisation, de façon à générer une population mixte de molécules de cADN bicaténaire,
c) la digestion de ladite population mixte de molécules de cADN bicaténaire par une enzyme de restriction spécifique dudit site de séquence de restriction 5',
d) le traitement de ladite population mixte de molécules de cADN bicaténaire par un agent de liaison, de sorte que des molécules de cADN bicaténaire individuelles soient liées les unes aux autres pour former une population mixte de concatémères,
e) l'exposition de ladite population mixte de concatémères à des amorces aléatoires dans des conditions d'amplification de génome total, de façon à générer l'acide nucléique amplifié,
f) la digestion au moins partielle dudit acide nucléique amplifié avec la même enzyme de restriction spécifique dudit site de séquence de restriction 5', générant ainsi une pluralité de séquences digérées, et
g) la liaison de séquences d'adaptation de séquençage aux extrémités de ladite pluralité de séquences digérées pour générer une population mixte de gabarits de séquençage liés à un adaptateur, lesdites séquences d'adaptation de séquençage contenant un site d'enzyme de restriction identique au site de séquence de restriction 5' dans le jeu d'amorces.

2. Procédé selon la revendication 1, dans lequel lesdites séquences d'adaptation de séquençage sont des séquences en épingle à cheveux.

3. Procédé selon la revendication 1, dans lequel ladite pluralité de séquences digérées comprend des séquences digérées individuelles qui contiennent chacune les séquences de base d'une seule desdites molécules de cADN bicaténaires parmi ladite population mixte de molécules de cADN bicaténaires.

4. Procédé selon la revendication 1, dans lequel ladite pluralité de séquences digérées comprend des séquences digérées individuelles qui contiennent chacune les séquences de base d'au moins deux desdites molécules de cADN bicaténaires parmi ladite population mixte de molécules de cADN bicaténaires, lesdites séquences de base étant séparées les unes des autres par lesdites séquences codes-barres.

5. Procédé selon la revendication 4, comprenant en outre le séquençage d'au moins une desdites séquences digérées individuelles pour générer des informations de séquence électronique, et le traitement desdites informations de séquence électronique par un algorithme d'alignement où lesdites séquences codes-barres servent à identifier des jonctions artificielles entre lesdites séquences de base d'au moins deux desdites molécules de cADN bicaténaires.

6. Procédé selon la revendication 1, dans lequel ladite séquence code-barres est identique dans chacune desdites amorces individuelles.

7. Procédé selon la revendication 1, dans lequel ledit gabarit ARN a une longueur inférieure à 2 000 bases.

8. Procédé selon la revendication 1, dans lequel ladite population mixte de concatémères comprend des concatémères individuels d'environ 2 000 bases de long ou plus.
